# EUROPEAN PATENT SPECIFICATION

(11) **EP 4 039 212 B1**
(45) Date of publication and mention of the grant of the patent: **21.01.2026**
(21) Application number: 22154241.8
(22) Date of filing: 31.01.2022
(51) Int. Cl.: A61B 18/14

(54) **TREATMENT DEVICE**
BEHANDLUNGSVORRICHTUNG
DISPOSITIF DE TRAITEMENT

(30) Priority: 03.02.2021 US 202163145237 P; 06.12.2021 US 202117542641
(43) Date of publication of application: 10.08.2022
(73) Proprietor: Olympus Medical Systems Corp., Tokyo 192-8507 (JP)
(72) Inventor: MIYAZAKI, Akira, Tokyo, 192-8507 (JP); ONUMA, Ryu, Tokyo, 192-8507 (JP); KITAMURA, Ojiro, Tokyo, 192-8507 (JP)
(74) Representative: Schicker, Silvia

(56) References cited:
- WO-A1-2018/054575
- WO-A2-2008/008457
- DE-T2- 60 210 880
- DE-U1- 202007 009 165
- US-A- 6 077 287
- US-A1- 2009 076 506
- US-A1- 2011 238 065
- US-A1- 2013 131 651
- US-A1- 2014 336 698

## Description

### RELATED APPLICATION DATA

This application is based on and claims priority under 35 U.S.C. §119 to U.S. Provisional Application No. 63/145,237, filed February 3, 2021.

### FIELD OF DISCLOSURE

The present disclosure relates to a treatment device having an openable-closable first and second jaws. The openable-closable jaws serve to clamp tissues for sealing and transecting. A blade is used to close the jaws and various mechanisms are disclosed for assisting the opening of the jaws, which is useful in the procedure of peeling tissues.

### BACKGROUND

In the discussion that follows, reference is made to certain structures and/or methods. However, the following references should not be construed as an admission that these structures and/or methods constitute prior art. Applicant expressly reserves the right to demonstrate that such structures and/or methods do not qualify as prior art against the present invention.

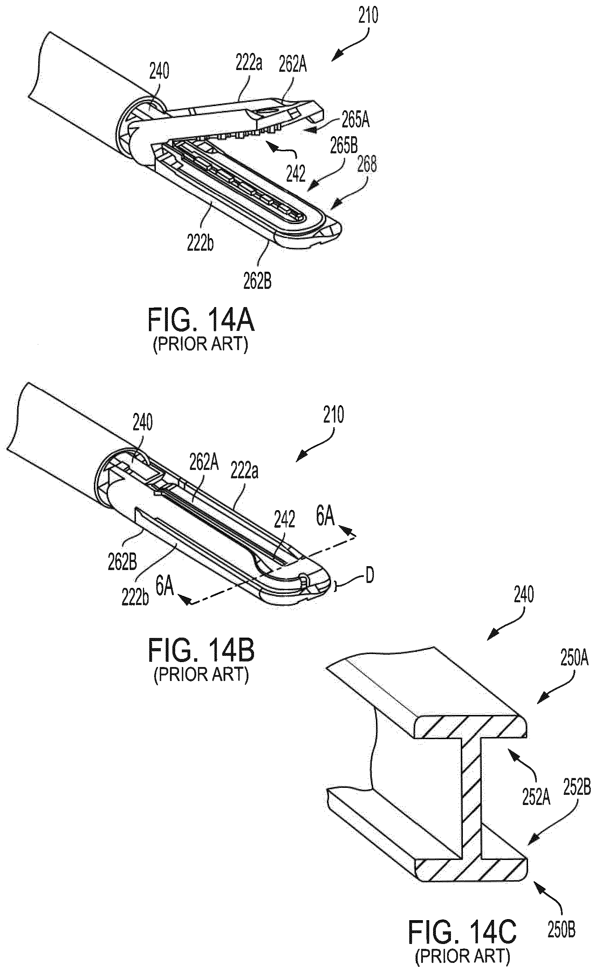

FIGS. 14A, 14B, and 14C are figures of a treatment device disclosed in the related art (United States Patent Application Publication No. 2009/0076506A1). FIG. 14A shows a working end 210 with jaws 222a and 222b in an opened position with outward-facing surfaces 262A and 262B and energy-delivery surfaces 265A and 265B. A channel 242 within the jaws accommodates the movement of reciprocating member 240, which may comprise a tissue-cutting element, for example by having a sharp distal edge. The edges 268 of energy-delivery surfaces 265A and 265B may be rounded to prevent the dissection of the tissues. FIG. 14B shows a working end 210 with jaws 222a and 222b in a closed position. The channel 242 where reciprocating member 240 is designed to move back and forth is visible, as well as dimension D between the energy-delivery surfaces. FIG. 14C shows the distal end of reciprocating member 240 having upper and lower flanges or "c"-shaped portions 250A and 250B. The flanges 250A and 250B respectively define inner cam surfaces 252A and 252B for slidably engaging outward-facing surfaces 262A and 262B of jaws 222a and 222b.

A drawback of the related art treatment device is that, although reciprocating member 240 serves to close the jaws 222a and 222b by sliding the reciprocating member 240 progressively forward through the channel 242, moving back the reciprocating member 240 provides a weak force to open the jaws 222a and 222b, which may not enough force to open the jaws 222a and 222b when there is a resistance. When the operators are required to open the jaws of the treatment device during the peeling procedure despite the resistance of any resisting objects, the related art treatment device would not be able to accomplish that task due to the lack of force for opening the jaws 222a and 222b.

DE 20 2007 009 165 U1 refers to a chirurgical instrument designed as a tubular shaft instrument. A shaft extends from a proximal end of the instrument on which a grip part is located. At its distal end, two clamping jaws as well as a bearing are arranged with a bearing bolt on which both the clamping jaws are mounted pivotably between an open- and a close position. Plural actuating devices are located on the grip part. Moreover, the chirurgical instrument comprises a separating device that is displaceable substantially parallel to the longitudinal axis of the instrument in a spacing located between the clamping jaws. The bearing bolt comprises a passage opening, which extends in a moving direction of the separating device. The separating device is guided from the proximal end to the area of the distal end of the clamping jaws in a kind of a groove in a guide, which is arranged on the clamping jaws in extension of and aligned with the passage opening.

US 6,077,287 A refers to a surgical instrument that includes a handle with an actuator assembly and a deflection control assembly. A proximal end portion of a rigid stem section is fixed to the handle. The rigid stem section includes a rigid main tube and an interface element fixed to a distal end portion of the main tube. The interface element has a disc-shaped main body portion. Upper and lower pairs of deflection control wire passages extend axially through a radially outer section of the main body portion of the interface element.

US 2011/0238065 A1 refers to arrangements of a surgical instrument designed to comprise an end effector having a cutting member. A handle is couplable to a proximal portion of an elongate shaft. The end effector is couplable to a distal portion of the elongate shaft. In the end effector, first and second jaws are operably couplable together. Regardless of how the first jaw and the second jaw are operably coupled together, they are configurable to rotate and/or deflect with respect to each other between an open position and a closed position when the cutting member translates with respect to the first jaw and/or the second jaw. When the end effector is in a closed configuration, the first jaw is in a curved configuration while gripping a tissue, and the cutting member is in a partially advanced position.

WO 2008/008457 A2 refers to an apparatus including forceps. The forceps include a jaw assembly that includes a pair of jaws. The forceps also include at least one blade for cutting tissue. In some arrangements, a single blade is disposed between the jaws. Channels are defined in each of the jaws to accommodate the blade.

DE 602 10 880 T2 refers to several different cutting device-retaining mechanisms that are usable to couple scissor blades to jaws when a scissor device is in a deployed position.

WO 2018/054575 A1 refers to a tubular shaft instrument with a distal, rotatable functional part.

US 2014/336698 A1 discloses an electrosurgical instrument that includes an end effector having jaws. A firing beam is longitudinally movable along part of the length of the end effector and includes a distal blade, an upper flange, and a lower flange. The "I-beam" type of configuration of firing beam provides closure of jaws as firing beam is advanced distally. The flange is configured to cam against a ramp feature at the proximal end of jaw to open jaw when firing beam 60 is retracted to a proximal position and to hold jaw open when firing beam remains at the proximal position. In some other versions, jaws are resiliently biased to an open position by a spring or other type of resilient feature.

US 2013/131651 A1 discloses an apparatus for operating on tissue includes an end effector having a first jaw and a second jaw. Upper jaw pivots relative to lower jaw via pivotal coupling positioned distal to ramped surface by actuating jaw cable. For example, jaw cable may be pushed distally to close upper jaw relative to lower jaw and jaw cable may be pulled proximally to open upper jaw relative to lower jaw. Jaw cable is configured to be rigid enough to translate upper jaw. In addition, blade member is translated by a blade cable. Actuation of jaw cable and blade cable may be staged. Namely, the trigger of the handle may be squeezed through a first range of motion to actuate jaw cable to close jaws, then the trigger may be squeezed through a second range of motion to actuate blade cable to drive blade.

### SUMMARY

Accordingly, there is a need for designing a treatment device with an improved structure to increase the forces that can be applied to open the jaws of the treatment end, which would substantially obviate one or more of the issues due to limitations and disadvantages of related art treatment device.

The invention is directed to the object to provide an improved treatment device that provides an efficient design for the opening and closing of the upper and lower jaws serving as high frequency electrodes compared to the related art. In order to address this object, a treatment device having the features defined in claim 1 is provided. Preferred embodiments are defined by the dependent claims.

In a first aspect, a treatment device according to the invention comprises a body including a movable handle, a longitudinally extending shaft having a proximal end connected to the body, and a treatment end pivotally joined to a distal end of the shaft. The treatment end includes a movable cutting blade, a jaw structure having a first jaw and a second jaw, and a first channel in the first jaw and a second channel in the second jaw. The first jaw and the second jaw are connected at a base end by a joint structure such that the first jaw is pivotable relative to the second jaw to open and close the jaw structure. When the jaw structure is in a closed state, the first channel and the second channel form a route for the cutting blade to move back and forth longitudinally between the base end of the jaw structure and a distal end of the jaw structure in response to a first input at the body. The treatment device further comprises means configured to apply a force to move the first jaw away from the second jaw in response to a second input at the body.

The treatment device comprises a wire configured to apply a force to pull the first jaw away from the second jaw. The wire is connected to one of the first jaw or the second jaw to apply the force to move the first jaw away from the second jaw, in response to the second input at the body. In particular, the means configured to apply the force to move the first jaw away from the second jaw in response to the second input at the body comprises a jaw pulling wire connected to one of the first jaw or the second jaw.

The second input described above may be a movement of the movable handle.

The body of the treatment device may include a connection to connect to a power source that is capable of supplying power for conducting a high-frequency treatment with the treatment device.

At least one of first and second jaws may be configured to serve as a high frequency electrode.

The movable cutting blade may be a different pole electrode compared to at least one of the first and second jaws.

A joint structure may be placed between the shaft and the first and second jaws.

The joint structure may include at least a pulley or a link.

In the treatment device according to the first aspect, the movable cutting blade may include a downward pushing surface and a upward pushing surface that are configured to close the jaw structure by sandwiching the first jaw and the second jaw while moving toward a distal side. In particular, the downward pushing surface and the upward pushing surface of the cutting blade may be configured interact so as to close the jaw structure by sandwiching the first jaw and the second jaw while the cutting blade moves toward a distal side.

The first jaw may be provided with a slope surface configured to contact the downward pushing surface.

The movable cutting blade is connected to a cutting blade member that is configured to transmit a driving force to open and/or close the jaw structure.

The treatment device further comprises a slider, which is placed between the movable handle and the first and second jaws.

The jaw pulling wire and the cutting blade member are connected to the slider.

The slider is linked to the movable handle.

In a second aspect, a treatment device comprises body including a movable handle, a longitudinal extending shaft having a proximal end connected to the body, and a treatment end pivotally joined to a distal end of the shaft. The treatment end includes a movable cutting blade, and a jaw structure having a first jaw and a second jaw, the first jaw and the second jaw being connected at a base end by a joint structure such that the first jaw is pivotable relative to the second jaw to open and close the jaw structure. The movable cutting blade includes a downward pushing surface and a upward pushing surface that are configured to close the jaw structure by sandwiching the first jaw and the second jaw while moving toward a distal side. The treatment device according to the second aspect, thus includes means configured to close the jaw structure.

The treatment device according to the second aspect also comprises means configured to apply a force to move the first jaw away from the second jaw, in response to a second input at the body. The means configured to apply the force to move the first jaw away from the second jaw in response to the second input at the body comprises a wire that is configured to apply a force to pull the first jaw away from the second jaw. The wire, specifically a jaw pulling wire, is connected to one of the first jaw or the second jaw.

Further, the movable cutting blade of the treatment device according to the second aspect is provided with a first channel in the first jaw and a second channel in the second jaw. The first channel and the second channel form a route for the cutting blade to move back and forth longitudinally between the base end of the jaw structure and a distal end of the jaw structure in response to a first input at the body, when the jaw structure is in a closed state.

In particular, in the treatment device according to the second aspect, the movable cutting blade is connected to a cutting blade member that is configured to transmit a driving force to close the jaw structure.

Further, in the treatment device according to the second aspect, the wire, in particular the jaw pulling wire, and the cutting blade member is connected to a slider. The slider is linked to the movable handle.

Further, in the treatment device according to the second aspect, the movable cutting blade is connected to a cutting blade member that is configured to transmit a driving force to open and/or close the jaw structure.

The treatment device according to the second aspect may be provided with further features described above with reference to the treatment device according to the first aspect, wherein these features may be combined as desired.

Other systems, features and advantages will be, or will become, apparent to one with skill in the art upon examination of the following figures and detailed description.

### BRIEF DESCRIPTION OF THE DRAWING

The following detailed description of preferred embodiments can be read in connection with the accompanying drawings in which like numerals designate like elements and in which:
FIG. 1 illustrates a treatment device including a body, a shaft, and a treatment end.
FIG. 2 illustrates an enlarged view of the treatment end shown in FIG. 1 and showing upper and lower jaws in an open configuration.
FIGS. 3A and 3 B illustrate a joint structure of the jaws and the joint structure between the treatment end and the shaft.
FIG. 4 illustrates in cross sectional view the upper and lower jaws of the treatment end and its internal structures.
FIG. 5 illustrates features of the upper jaw and an upper channel used to move an I-shaped cutting blade.
FIG. 6 illustrates the cross-sectional schema of the upper and lower jaws of the treatment end in its closed state.
FIGS. 7A and 7B illustrates in cross sectional view the upper and lower jaws of the treatment end and its internal structures.
FIG. 8A illustrates features of the inner view of the upper jaw and the upper channel used to move the I-shaped cutting blade.
FIG. 8B illustrates, in a back-perspective view, features of the upper and lower jaws and the structures used for opening the upper jaw.
FIG. 9A illustrates in cross sectional view the upper and lower jaws of the treatment end in an opened state.
FIG. 9B illustrates features of the upper and lower jaws and the structures used for opening the upper jaw.
FIG. 10 illustrates features of the upper and lower jaws and the structures used for opening the upper jaw.
FIGS. 11A, 11B, and 11C illustrates the upper and lower jaws in open and closed positions (FIG. 11A and FIGS. 11B and 11C, respectively) and structures used for closing the upper jaw.
FIG. 12 illustrates the cross-sectional schema of the upper and lower jaws of the treatment end in its closed state.
FIGS. 13A, 13B, and 13C illustrates the upper and lower jaws in closed and open positions (FIG. 13A and FIGS. 13B and 13C, respectively) and structures used for opening the upper jaw.
FIGS. 14A to 14C are figures of a treatment device disclosed in the related art.

For ease of viewing, in some instances only some of the named features in the figures are labeled with reference numerals.

### DETAILED DESCRIPTION

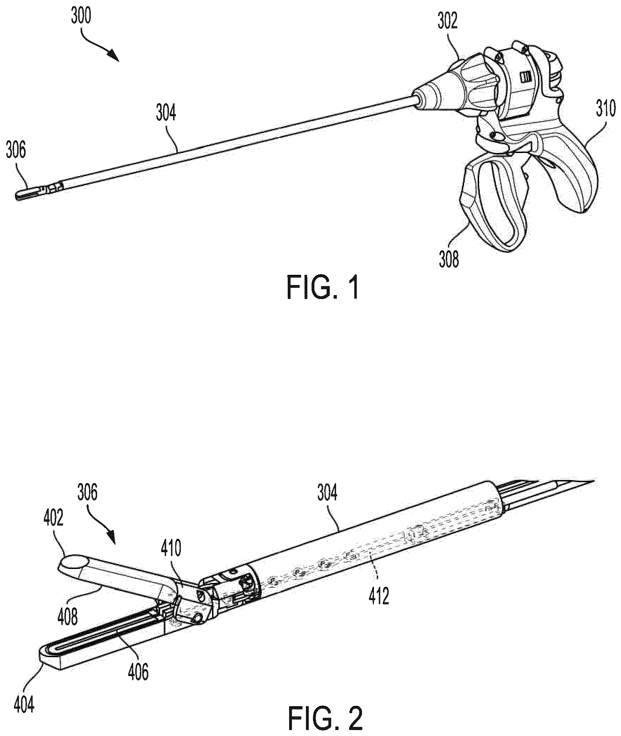

FIG. 1 is an illustration of a treatment device 300 including a body 302, a shaft 304, and a treatment end 306. The body 302 includes a moving arm 308 and a grip 310. The moving arm 308 is used to actuate and operate the functions of treatment end 306. The grip 310 is connected to a power source supplying power used for high-frequency treatment of treatment device 300. The power source can be a wired or wireless power source. The shaft 304 protects the wires and members within, necessary for operating the functions of treatment end 306.

FIG. 2 is an illustration of the treatment end 306 including an upper jaw 402 and lower jaw 404. The upper jaw 402 and lower jaw 404 may serve as electrodes electrically conducting the power supplied from the body 302 through the shaft 304. When the upper jaw 402 and lower jaw 404 are in a closed position, high frequency currents would be conducted between the jaws, and any object clasped between the jaws would either be dissected or sealed using the conducted high-frequency currents.

Both the upper jaw 402 and lower jaw 404 includes channels for moving a cutting blade 410, which may also serve as a high frequency electrode. An upper channel 406 and a lower channel 408 are used as a route for the cutting blade 410 to move back and forth longitudinally between a base end of the jaws, i.e., the end connected for pivoting the jaws open and closed, and a distal end of the jaws. Moving the cutting blade 410 back and forth in the channels also serves to open and close the jaws, such as by displacing the upper jaw 402 relative to the lower jaw 404, which may be stationary attached to the body of the treatment end 306. An operation member 412 goes through the shaft 304 and is used for operating the functions of the treatment end 306. The operation member 412 can take any suitable form, such as cables and pulleys or other connection system that transmits input to control the operation of features of the treatment device 300, in particular features associated with the treatment end 306.

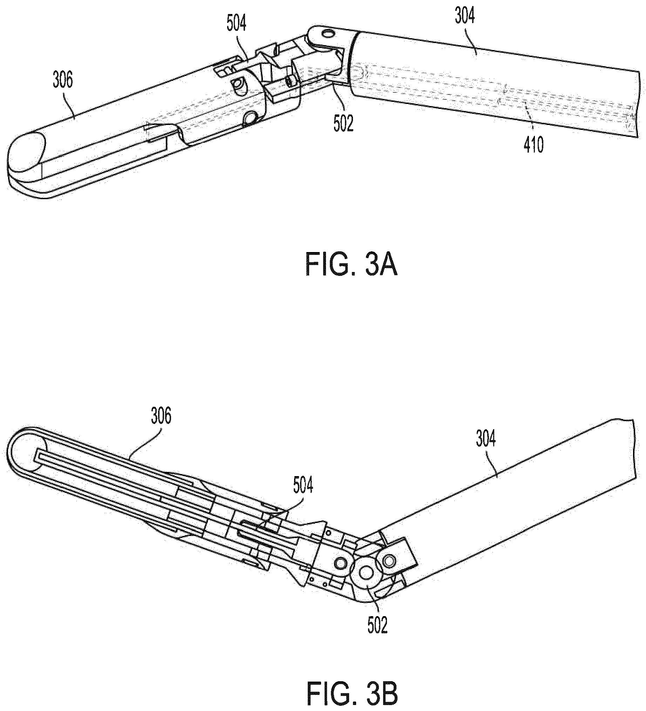

FIGS. 3A and 3B illustrate a joint structure 502 that is formed between shaft 304 and treatment end 306. The joint structure 502 serves to enable angular manipulation of the treatment end 306, enhancing the usability of the treatment device 300. The downside of having the joint structure 502 is that the added complexity in the structure serves as obstacles for the operation member 412 to pass through and effectively convey the operation movements to the treatment device 300. FIGS. 3A and 3B also illustrate features of a joint structure 504 of the jaw, which allow opening and closing of the jaws during operation.

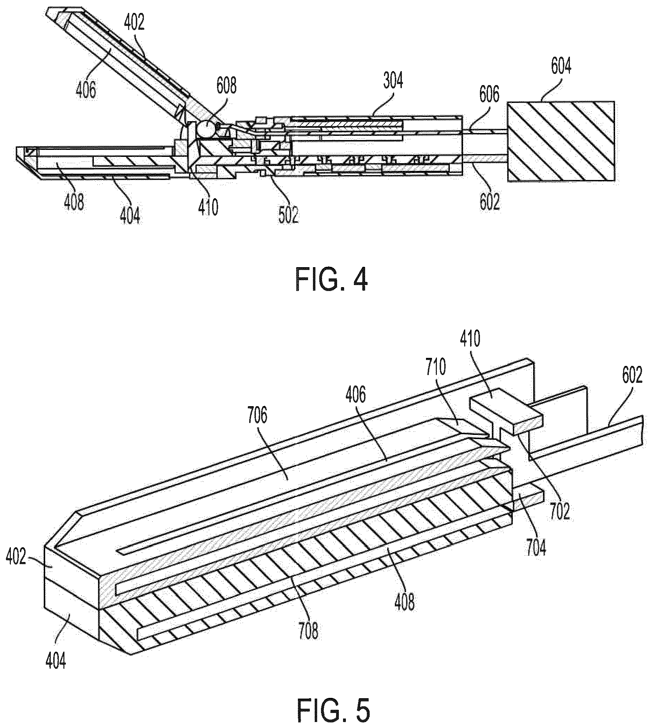

FIG. 4 illustrates in cross sectional view the upper and lower jaws of the treatment end 306 and its internal structures, including the mechanism for opening and closing the upper jaw 402 and lower jaw 404. The cutting blade 410 is connected to a cutting blade member 602 that goes through shaft 304 and connects to a slider 604. The slider 604 is operable through movable arm 308 or other operating structures such as motor mechanisms. When the upper jaw 402 and lower jaw 404 are closed, the upper channel 406 and the lower channel 408 form a space in which the cutting blade 410 moves as the cutting blade member 602 is progressively pushed by operation of the slider 604. The cutting blade 410 travels within the space and can traverse back and forth between a first position adjacent the base end of the jaws and a second position at a distal end of the jaws. When there is an object located in the noted space between the upper jaw 402 and lower jaw 404, the cutting blade 410 traversing the upper channel 406 and the lower channel 408 serves to dissect such object using its blade formed on its distal end. The cutting blade 410 also has structures that cooperate with features of the upper channel 406 and the lower channel 408 to operate to close the upper jaw 402 using the structural design of the upper channel 406, which is further described herein. The upper jaw is connected to an upper jaw pulling wire 606, which is also connected to the slider 604. In response to the operation of the slider 604 to pull the upper jaw pulling wire 606, the upper jaw 402 is pulled away from the lower jaw 404 using a rotating fulcrum 608 placed at the base end of the upper jaw 402. The pulling of the upper jaw pulling wire 606 would occur simultaneously with pulling of the cutting blade member 602, which serves to pull back the cutting blade 410.

FIG. 5 is an inner view illustration of the upper jaw 402 and lower jaw 404. The I-shaped cutting blade 410 has a downward pushing surface 702 and an upward pushing surface 704 connected to the cutting blade member 602. The downward pushing surface 702 and the upward pushing surface 704 come in contact with a corresponding upper surface 706 and lower surface 708 of the upper and lower channels respectively when the cutting blade 410 goes through said channels. The downward pushing surface 702 initially contacts sloped surface 710 placed at the proximal end of the upper surface 706 and gradually slides past the sloped surface 710, thereby gradually pushing down the upper jaw 402 towards the lower jaw 404 to cause the upper jaw 402 to move toward the lower jaw 404. Although upward, downward, upper and lower are used herein, it is readily understood that these terms are in relation to the orientation depicted in the drawings and that a change in orientation does not change the position, operation and interoperability of features so described.

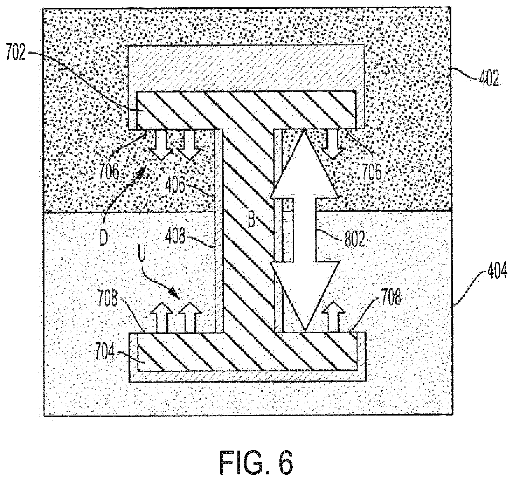

FIG. 6 is a cross sectional schema of the upper jaw 402 and lower jaw 404 where the jaws are in the closed state. A height 802 of a blade portion B of the I-shaped cutting blade 410 is equivalent (within manufacturing tolerances) to the distance between the upper surface 706 of the upper channel 406 and the lower surface 708 of the lower channel 408 when the upper jaw 402 and lower jaw 404 are in their closed positions. When starting with the jaws in the open state, pushing the cutting blade 410 through the space formed by the lower channel 406 and upper channel 408 towards the distal end of the treatment end 306 pushing surface 702 applies a force on the upper surface 706 in a downward direction, i.e., depicted by arrows D. Relatedly, pushing surface 704 applies a force on the lower surface 708 in an upward direction, i.e., depicted by arrow U. The arrows D and U on both sides of the blade portion B of the I-shaped cutting blade 410 is indicative that those forces can be applied on both sides of the central portion. In some embodiments, the lower jaw 404 is fixed and non-movable relative to the body of the treatment end 306 and/or the shaft 304, and the upper jaw 402 would be pushed downwards towards the lower jaw 404 using the rotating fulcrum 608 placed at the base end of the upper jaw 402 until the two jaws are closed together. In other embodiments, the upper jaw 402 is fixed and non-movable relative to the body of the treatment end 306 and/or the shaft 304, and the lower jaw 404 would be pushed upwards towards the upper jaw 402 using the rotating fulcrum 608 placed at the base end of the lower jaw 404 until the two jaws are closed together.

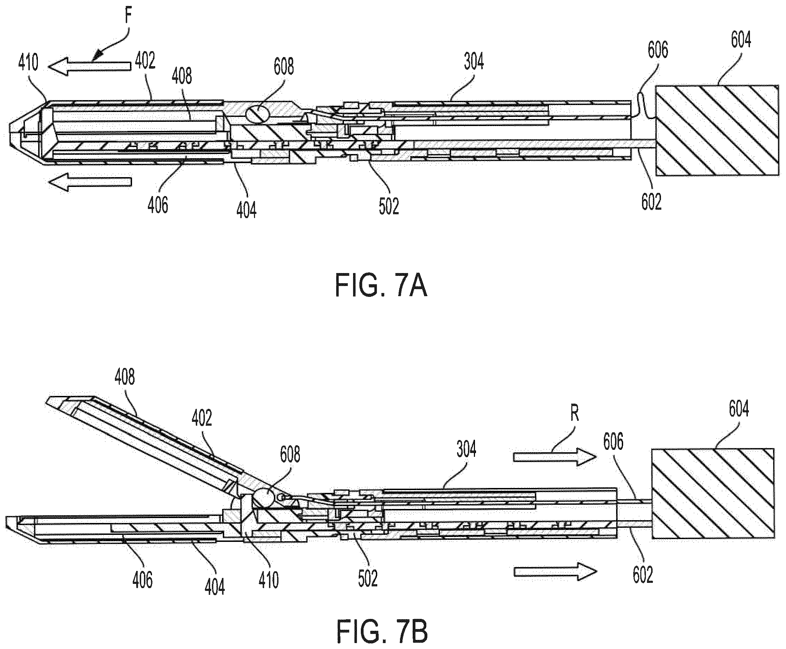

FIGS. 7A and 7B further illustrate the mechanism of opening and closing of upper jaw 402 relative to lower jaw 404. As shown in FIG. 7A, when the slider 604 pushes the cutting blade member 602, cutting blade 410 would be pushed forward from proximal end towards the distal end (as shown by arrow) of the treatment end 306 through the upper and lower channels, closing upper jaw 402 and the lower jaw 404. The downward pushing surface 702 initially contacts sloped surface 710 and gradually slides through the sloped surface 710 and the upper surface 704 by pushing downward the upper jaw 402 towards the lower jaw 404. The upper jaw pulling wire 606 is also simultaneously pushed into the shaft 304 using slider 604, forming loose tensions in the upper jaw pulling wire 606. As shown in FIG. 7B, when the slider 604 pulls back the cutting blade member 602, cutting blade 410 would be pulled back through the upper and lower channels from the distal end towards the proximal end of the treatment end 306 and is retracted (as shown by arrow R). The upper jaw pulling wire 606 will be simultaneously pulled back by the slider 604, opening the upper jaw 402 using the rotating fulcrum 608 placed at the base end of the upper jaw 402. Through the continued pulling back force through the pulling wire 606, sufficient opening strength of the upper jaw 402 would be realized, enabling the peeling functions required for the operator during the treatment process.

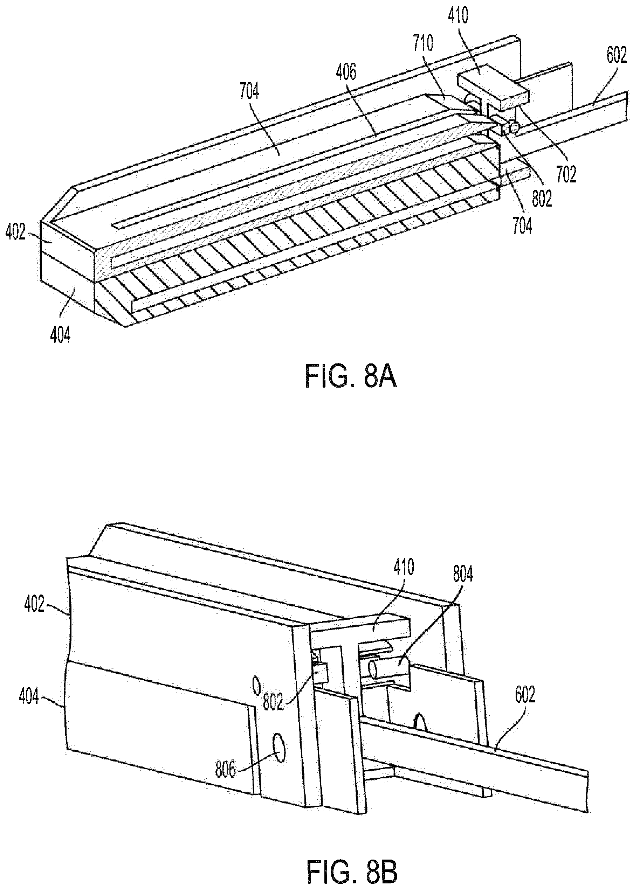

FIG. 8A and 8B illustrates an example, which does not form part of the present invention but contains information useful for understanding the present invention, illustrating the mechanism of opening and closing of upper jaw 402 relative to lower jaw 404. As shown in FIG. 8A, when the cutting blade member 602 is pushed forward towards the distal end of the treatment end 306, the downward pushing surface 702 of the cutting blade 410 would push downward the upper surface 706, thereby closing the upper jaw 402 toward the lower jaw 404, in case the upper jaw 402 was in the open state. The downward pushing surface 702 initially contacts sloped surface 710 and gradually slides through the sloped surface 710 and the upper surface 704 by pushing the upper jaw 402 downwards towards the lower jaw 404. On the other hand, a hooking member 802 protruding from the cutting blade 410 would go through a channel formed beneath the upper surface 702, the channel being part of the upper channel 406. As shown in FIG. 8B, when the cutting blade 410 is pulled backward by the cutting blade member 602, a protruding member 804 being part of the upper jaw 402 comes in contact with the hooking member 802. The pulling back force of the cutting blade 410 together with the rotating motion achieved by a rotating fulcrum 806 placed at the lower jaw 404 would serve to rotate open the upper jaw 402 into the open state. Through the continued pulling back force through the cutting blade member 602, sufficient opening strength of the upper jaw 402 would be realized, enabling the peeling functions required for the operator during the treatment process. By coming in contact with the protruding member 804, the hooking member 802 would also stop the cutting blade 410 from pulling back too much into shaft 304. The resistance of the protruding member 804 felt through the movable handle 308 would inform the operator that the cutting blade 410 has reached the end point of its pulling back and would also inform the operator that the opening of the jaw 402 would start.

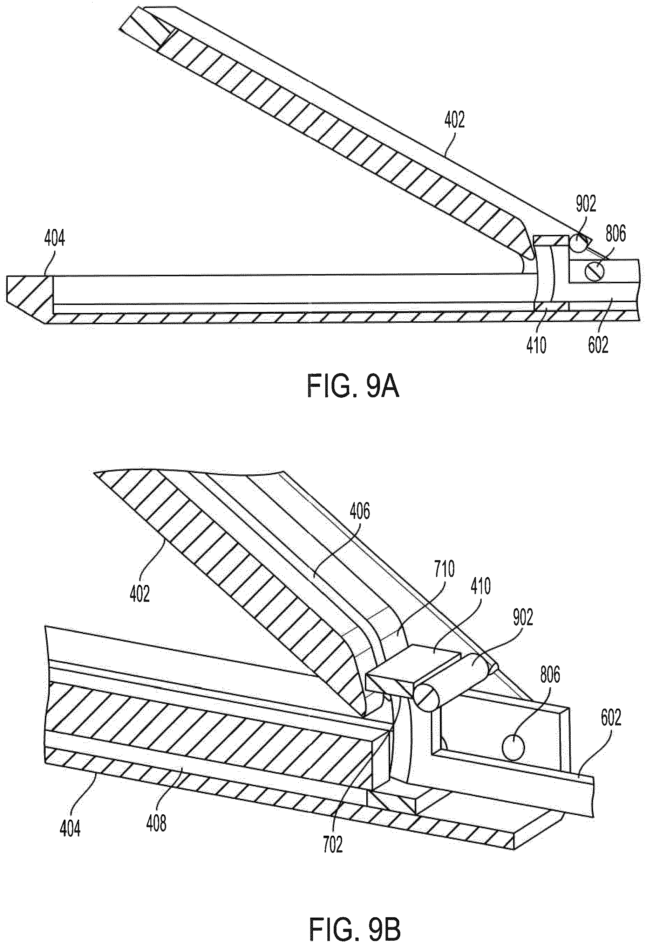

FIG. 9A and 9B illustrates an example, which does not form part of the present invention but contains information useful for understanding the present invention, further illustrating the mechanism of opening and closing of upper jaw 402 relative to lower jaw 404. As shown in FIG. 9A, when the cutting blade member 602 is pushed backward towards the proximal end of the treatment end 306, an upper portion of the cutting blade 410 would make contact with a protruding member 902. Further pulling backward the cutting blade 410 together with the rotating motion achieved by the rotating fulcrum 806 placed at the lower jaw 404 would serve to rotate open the upper jaw 402 into the open state. Through the continued pulling back force through the cutting blade member 602, sufficient opening strength of the upper jaw 402 would be realized, enabling the peeling functions required for the operator during the treatment process.

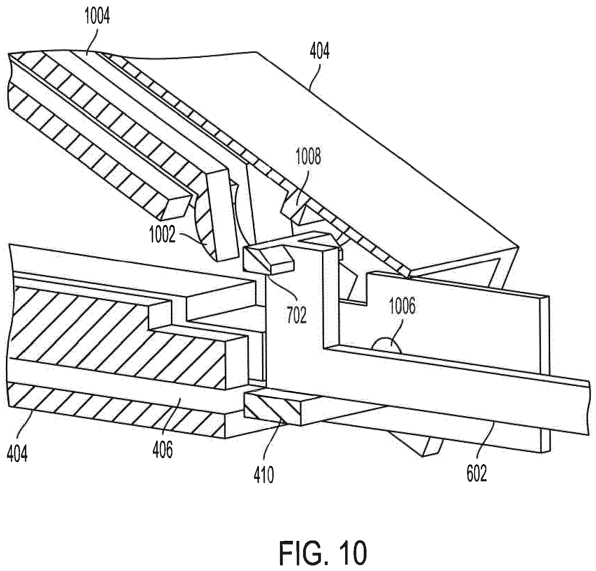

FIG. 10 illustrates an example, which does not form part of the present invention but contains information useful for understanding the present invention, illustrating the mechanism of opening and closing of upper jaw 402 relative to lower jaw 404. The cutting blade 410 is pushed forward by the cutting blade member 602 through the upper and lower channels. The downward pushing surface 702 of the cutting blade 410 contacts a sloped surface 1002 and gradually pushes down a movable upper channel 1004. Because the movable upper channel 1004 is connected to the upper jaw 404, the downward force, together with the rotation movement of rotating fulcrum 1006, would push the upper jaw 402 downwards towards its closed state. A stopper 1008 serves to stop the movable upper channel 1004 from being pulled back towards the proximal direction of the treatment device 300 when the cutting blade 410 is pulled back for opening the upper jaw 402, as further described below.

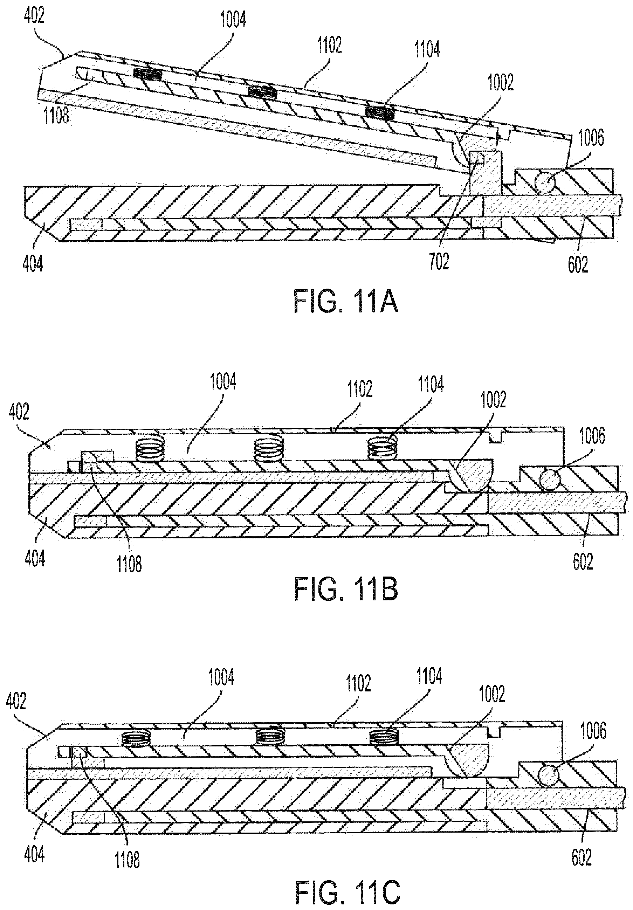

FIG. 11A, 11B, and 11C illustrates a cross sectional view of the upper and lower jaws and the mechanism of closing the upper jaw 402 relative to lower jaw 404 using a displaceable upper channel 1004. As shown in FIG. 11A, when the downward pushing surface 702 of the cutting blade 410 contacts and pushes downward the slope 1002, the displaceable upper channel 1004 receives a downward force. Because the displaceable upper channel 1004 is connected to a ceiling 1102 of upper jaw 402 through elastic members 1104, the entire upper jaw 402 will be moved downward using the rotation movement of the rotating fulcrum 1006. The displaceable upper channel 1004 will be pulled downward within the upper jaw 402 using the expanding nature of the elastic members 1104, separated from the ceiling 1104. The elastic member 1104 can be a spring, a rubber band, or other elastic member allowing elongation and contraction. As shown in FIG. 11B, the displaceable upper channel 1004 would be pushed downward by the downward pushing surface 702 of the cutting blade 410, all the while the elastic member 1104 maintains the contracting force conveying upward bias to the movable upper channel 1004. As shown in FIG. 11C, when the downward pushing surface 702 of the cutting blade 410 reaches the distal end of the treatment end 306 and an opening 1108 of the displaceable upper channel 1004, the displaceable upper channel 1004 would be pulled upward by the contraction of the elastic member 1104 towards the ceiling 1102.

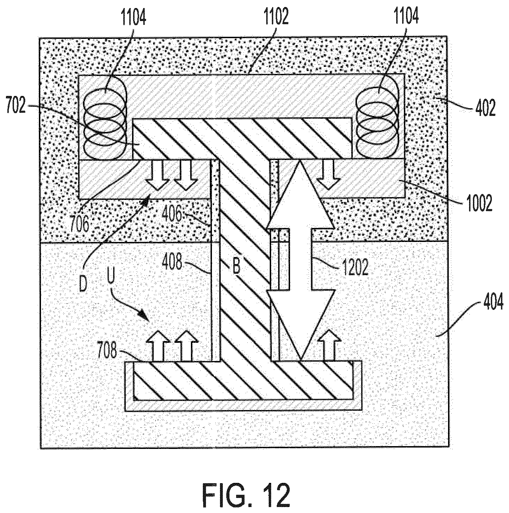

FIG. 12 illustrates the cross-sectional view of the upper and lower jaws in a closed state. A height 1202 of the blade portion B of the I-shaped cutting blade 410 is equivalent (within manufacturing tolerances) to the distance between the upper surface 706 of the upper channel 406 and the lower surface 708 of the lower channel 408 when the upper jaw 402 and lower jaw 404 are in their closed positions. The downward pushing surface 702 of the I-shaped cutting blade 410 pushes down the displaceable upper channel 1004, separating it away from the ceiling 1102 of the upper jaw 402. The elastic member 1104 would be expanded but maintains the contraction force that pulls the movable upper channel upward in the direction of the ceiling 1102.

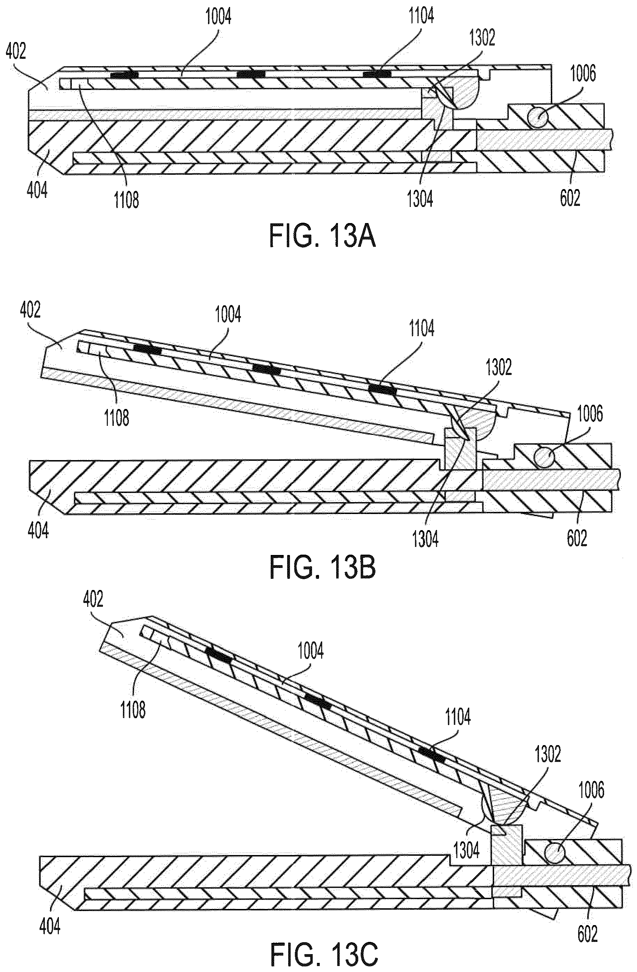

FIGS. 13A, 13B, and 13C illustrate the mechanism of opening the upper jaw 402 relative to lower jaw 404 using the displaceable upper channel 1004. As shown in FIG. 13A, when the cutting blade 410 is pulled backward away from the distal end of the treatment end 306, it will slide under the displaceable upper channel 1004 using the opening 1108 and a cutting blade sloped surface 1302 formed at the upper surface of the cutting blade 410. The cutting blade 410 would then slide beneath the displaceable upper channel 1004 until the cutting blade slope 1302 comes in contact with a curved surface 1304, which is part of the movable upper channel 1004. The curved surface can have any suitable curved shape, such as a semicircle, an oval or an ovid, both partial and full semicircle, oval and ovid shapes can be used. As shown in FIG. 13B, the cutting blade slope 1302 would contact and apply a force to the curved surface 1304, thereby pushing the displaceable channel 1004 upward. The entire upper jaw 402 would open upward as a result of such application of upward force and the rotation movement of the rotating fulcrum 1006. As shown in FIG. 13C, as the cutting blade 410 continues pulling backward in the proximal direction, the cutting blade slope 1302 would slide past the curved surface 1304 of the displaceable upper channel 1004 and the two parts would exchange its position. The position of the displaceable upper channel 1002 and the cutting blade 410 would revert back to pre-closing state described in FIG. 10A. Through the force resulting from the continued movement of the cutting blade member 602, sufficient opening force is applied to the upper jaw 402, enabling the peeling functions required for the operator during the treatment process.

## Claims

1. A treatment device (300), comprising:
a body (302) including a movable handle (308);
a slider (604) configured to move based on an operation on the movable handle (308);
a longitudinally extending shaft (304) having a proximal end connected to the body (302); and
a treatment end (306) pivotally joined to a distal end of the shaft (304),
wherein the treatment end (306) includes:
a movable cutting blade (410),
a jaw structure having a first jaw (402) and a second jaw (404), the first jaw (402) and the second jaw (404) being connected at a base end by a joint structure (504) such that the first jaw (402) is pivotable relative to the second jaw (404) to open and close the jaw structure,
a first channel (406) in the first jaw (402) and a second channel (408) in the second jaw (404), wherein with the jaw structure in a closed state, the first channel (406) and the second channel (408) form a route for the cutting blade (410) to move back and forth longitudinally between the base end of the jaw structure and a distal end of the jaw structure, in response to a first input at the body (302), and
means configured to apply a force to move the first jaw (402) away from the second jaw (404) in response to a second input at the body (302); and
wherein the treatment device (300) is configured such that:
- a first operation on the movable handle (308) closes the first jaw (402) and the second jaw (404) and pushes the movable cutting blade (410) out;
- a second operation on the movable handle (308) opens the first jaw (402) and the second jaw (404) and retracts the movable cutting blade (410);
and
- in the second operation, the first jaw (402) is pulled toward a base end side in response to the slider movement, which causes the first jaw (402) and the second jaw (404) to open as the first jaw (402) rotate around a rotating fulcrum (608),
wherein the means configured to apply the force to move the first jaw (402) away from the second jaw (404) in response to the second input at the body (302) comprises a jaw pulling wire (606) connected to one of the first jaw (402) or the second jaw (404),
wherein the jaw pulling wire (606) forms loose tensions in the first operation,
wherein the movable cutting blade (410) is connected to a cutting blade member (602) that is configured to transmit a driving force to close the jaw structure;
wherein the slider (604) is placed between the movable handle (308) and the first and second jaws (402, 404),
the jaw pulling wire (606) and the cutting blade member (602) are connected to the slider (604), the slider (604) being linked to the movable handle (308).

2. The treatment device (300) according to claim 1,
- wherein the second input is a movement of the movable handle (308); and/or
- wherein the body (302) includes a connection to connect to a power source that is capable of supplying power for conducting a high-frequency treatment with the treatment device (300), and wherein at least one of the first jaw (402) and second jaw (404) is configured to serve as a high frequency electrode; and/or
- wherein the movable cutting blade (410) is a different pole electrode compared to at least one of the first and second jaws (402, 404).

3. The treatment device (300) according to claim 1, wherein a joint structure (502) is placed between the shaft (304) and the first and second jaws (402, 404).

4. The treatment device (300) according to claim 3, wherein the joint structure (502) includes at least a pulley or a link.

5. The treatment device (300) according to claim 1, wherein the movable cutting blade (410) includes:
a downward pushing surface (702) and a upward pushing surface (704) that are configured to close the jaw structure by sandwiching the first jaw (402) and the second jaw (402) while moving toward a distal side.

6. The treatment device (300) according to claim 10, wherein the first jaw (402) is provided with a slope surface (710) configured to contact the downward pushing surface (702).

## Patentansprüche

1. Behandlungsvorrichtung (300), die umfasst:
einen Körper (302) mit einem bewegbaren Griff (308);
einen Schieber (604), der konfiguriert ist, sich auf Grundlage einer Betätigung an dem beweglichen Griff (308) zu bewegen;
einen sich in Längsrichtung erstreckenden Schaft (304) mit einem proximalen Ende, das mit dem Körper (302) verbunden ist; und
ein Behandlungsende (306), das schwenkbar mit einem distalen Ende des Schafts (304) verbunden ist,
wobei das Behandlungsende (306) umfasst:
eine bewegbare Schneidklinge (410),
eine Backenstruktur mit einer ersten Backe (402) und einer zweiten Backe (404), wobei die erste Backe (402) und die zweite Backe (404) an einem Basisende durch eine Gelenkstruktur (504) verbunden sind, so dass die erste Backe (402) relativ zu der zweiten Backe (404) schwenkbar ist, um die Backenstruktur zu öffnen und zu schließen,
einen ersten Kanal (406) in der ersten Backe (402) und einen zweiten Kanal (408) in der zweiten Backe (404), wobei in einem geschlossenen Zustand der Backenstruktur der erste Kanal (406) und der zweite Kanal (408) einen Weg für die Schneidklinge (410) bilden, um sich in Reaktion auf eine erste Eingabe an dem Körper (302) in Längsrichtung zwischen dem Basisende der Backenstruktur und einem distalen Ende der Backenstruktur hin und her zu bewegen, und
eine Einrichtung, die konfiguriert ist, eine Kraft auszuüben, um in Reaktion auf eine zweite Eingabe an dem Körper (302) die erste Backe (402) von der zweiten Backe (404) weg zu bewegen; und
wobei die Behandlungsvorrichtung (300) so konfiguriert ist, dass:
- eine erste Betätigung an dem beweglichen Griff (308) die erste Backe (402) und die zweite Backe (404) schließt und die bewegliche Schneidklinge (410) herausdrückt;
- eine zweite Betätigung an dem beweglichen Griff (308) die erste Backe (402) und die zweite Backe (404) öffnet und die bewegliche Schneidklinge (410) zurückzieht; und
- bei der zweiten Betätigung die erste Backe (402) in Reaktion auf die Schieberbewegung in Richtung einer Basisendseite gezogen wird, wodurch sich die erste Backe (402) und die zweite Backe (404) öffnen, wenn sich die erste Backe (402) um einen Drehpunkt (608) dreht,
wobei die Einrichtung, die konfiguriert ist, eine Kraft auszuüben, um die erste Backe (402) in Reaktion auf die zweite Eingabe an dem Körper (302) von der zweiten Backe (404) weg zu bewegen, einen Backenzugdraht (606) umfasst, der mit der ersten Backe (402) oder der zweiten Backe (404) verbunden ist,
wobei der Backenzugdraht (606) lockere Spannungen bei der ersten Betätigung bildet,
wobei die bewegbare Schneidklinge (410) mit einem Schneidklingenelement (602) verbunden ist, das konfiguriert ist, eine Antriebskraft zum Schließen der Backenstruktur zu übertragen;
wobei der Schieber (604) zwischen dem bewegbaren Griff (308) und den ersten und zweiten Backen (402, 404) angeordnet ist,
der Backenzugdraht (606) und das Schneidklingenelement (602) mit dem Schieber (604) verbunden sind, wobei der Schieber (604) mit dem bewegbaren Griff (308) verbunden ist.

2. Behandlungsvorrichtung (300) gemäß Anspruch 1,
- wobei die zweite Eingabe eine Bewegung des bewegbaren Griffs (308) ist; und/oder
- wobei der Körper (302) eine Verbindung zum Anschluss an eine Energiequelle umfasst, die in der Lage ist, Energie für die Durchführung einer Hochfrequenzbehandlung mit der Behandlungsvorrichtung (300) zu liefern, und wobei die erste Backe (402) und/oder die zweite Backe (404) konfiguriert ist, als Hochfrequenzelektrode zu dienen; und/oder
- wobei die bewegbare Schneideklinge (410) im Vergleich zu mindestens einer der ersten und zweiten Backen (402, 404) eine andere Polelektrode.

3. Behandlungsvorrichtung (300) gemäß Anspruch 1, wobei eine Gelenkstruktur (502) zwischen dem Schaft (304) und den ersten und zweiten Backen (402, 404) angeordnet ist.

4. Behandlungsvorrichtung (300) gemäß Anspruch 3, wobei die Gelenkstruktur (502) zumindest eine Riemenscheibe oder ein Verbindungsglied umfasst.

5. Behandlungsvorrichtung (300) gemäß Anspruch 1, wobei die bewegbare Schneidklinge (410) umfasst:
eine nach unten drückende Fläche (702) und eine nach oben drückende Fläche (704), die so konfiguriert sind, dass sie die Backenstruktur schließen, indem sie die erste Backe (402) und die zweite Backe (402) einklemmen, während sie sich in Richtung einer distalen Seite bewegen.

6. Behandlungsvorrichtung (300) gemäß Anspruch 10, wobei die erste Backe (402) mit einer geneigten Fläche (710) versehen ist, die so konfiguriert ist, dass sie mit der nach unten drückenden Fläche (702) in Kontakt kommt.

## Revendications

1. Dispositif de traitement (300) comprenant:
un corps (302) comprenant une poignée mobile (308);
un coulisseau (604) conçu pour se déplacer en fonction d'un actionnement effectué sur la poignée mobile (308);
une tige s'étendant longitudinalement (304) ayant une extrémité proximale reliée au corps (302); et
un embout de traitement (306) relié de manière pivotante à une extrémité distale du tube (304),
dans lequel l'embout de traitement (306) comprend:
une lame de coupe mobile (410),
une structure à mâchoires comportant une première mâchoire (402) et une deuxième mâchoire (404), la première mâchoire (402) et la deuxième mâchoire (404) étant reliées à leur extrémité de base par une structure à charnière (504) de telle sorte que la première mâchoire (402) puisse pivoter par rapport à la deuxième mâchoire (404) afin d'ouvrir et de fermer la structure à mâchoires,
un premier canal (406) dans la première mâchoire (402) et un deuxième canal (408) dans la deuxième mâchoire (404), dans lequel, lorsque la structure à mâchoires se ferme, le premier canal (406) et le deuxième canal (408) forment un chemin permettant à la lame de coupe (410) de se déplacer longitudinalement dans un mouvement de va-et-vient entre l'extrémité de base de la structure à mâchoires et une extrémité distale de la structure à mâchoires, en réponse à une première entrée au niveau du corps (302), et
un moyen conçu pour appliquer une force afin d'éloigner la première mâchoire (402) de la deuxième mâchoire (404) en réponse à une deuxième entrée au niveau du corps (302); et
dans lequel le dispositif de traitement (300) est conçu de telle sorte que:
- un premier actionnement sur la poignée mobile (308) ferme la première mâchoire (402) et la deuxième mâchoire (404) et pousse la lame de coupe mobile (410) vers l'extérieur;
- un deuxième actionnement sur la poignée mobile (308) ouvre la première mâchoire (402) et la deuxième mâchoire (404) et rétracte la lame de coupe mobile (410); et
- lors du deuxième actionnement, la première mâchoire (402) est tirée vers un côté d'extrémité de base en réponse à un mouvement du coulisseau, ce qui amène la première mâchoire (402) et la deuxième mâchoire (404) à s'ouvrir lorsque la première mâchoire (402) pivote autour d'un axe de pivotement (608),
dans lequel le moyen conçu pour appliquer la force afin d'éloigner la première mâchoire (402) de la deuxième mâchoire (404) en réponse à la deuxième entrée au niveau du corps (302) comprend un fil de traction de mâchoire (606) relié à l'une parmi la première mâchoire (402) et la deuxième mâchoire (404),
dans lequel le fil de traction de mâchoire (606) forme des tensions lâches lors du premier actionnement,
dans lequel la lame de coupe mobile (410) est reliée à un élément de lame de coupe (602) qui est conçu pour transmettre une force d'entraînement afin de fermer la structure à mâchoires;
dans lequel le coulisseau (604) est placé entre la poignée mobile (308) et les première et deuxième mâchoires (402, 404),
le fil de traction de mâchoire (606) et l'élément de lame de coupe (602) sont reliés au coulisseau (604), le coulisseau (604) étant relié à la poignée mobile (308).

2. Dispositif de traitement (300) selon la revendication 1,
- dans lequel la deuxième entrée est un mouvement de la poignée mobile (308); et/ou
- dans lequel le corps (302) comprend une connexion pour se connecter à une source d'alimentation pouvant fournir de l'énergie pour effectuer un traitement à haute fréquence à l'aide du dispositif de traitement (300), et dans lequel au moins l'une parmi la première mâchoire (402) et la deuxième mâchoire (404) est conçue pour servir d'électrode à haute fréquence; et/ou
- dans lequel la lame de coupe mobile (410) est une électrode de pôle différente par rapport à au moins l'une parmi les première et deuxième mâchoires (402, 404).

3. Dispositif de traitement (300) selon la revendication 1, dans lequel une structure à charnière (502) est placée entre la tige (304) et les première et deuxième mâchoires (402, 404).

4. Dispositif de traitement (300) selon la revendication 3, dans lequel la structure à charnière (502) comprend au moins une poulie ou une biellette.

5. Dispositif de traitement (300) selon la revendication 1, dans lequel la lame de coupe mobile (410) comprend:
une surface de poussée vers le bas (702) et une surface de poussée vers le haut (704) qui sont conçues pour fermer la structure à mâchoires en intercalant la première mâchoire (402) et la deuxième mâchoire (402) tout en se déplaçant vers un côté distal.

6. Dispositif de traitement (300) selon la revendication 10, dans lequel la première mâchoire (402) est dotée d'une surface en pente (710) conçu pour entrer en contact avec la surface de poussée vers le bas (320, 702).
